# EUROPEAN PATENT APPLICATION

(11) **EP 0 581 972 A1**
(43) Date of publication of application: **09.02.1994**
(21) Application number: 92106448.1
(22) Date of filing: 15.04.1992
(51) Int. Cl.: A61K 33/04

(54) **Hemorrhoidal composition flaves of sulphur and cream of tartar**

(71) Applicant: Verde, Giancarlo U., Rome (IT)
(72) Inventor: The designation of the inventor has not yet been filed

(57) **Abstract**

A composition comprising 22 weight percent sulfur(is better to use the''flowers of sulfur'') and 78 weight percent cream of tartar.

The invention is also relates to therapeutic method by ingesting a advised effective but not laxative dose of this composition without any collateral effects.

The composition is useful for the elimination and the treatment of hemorrhoids and hemorrhoidal symptoms.

## Description

### BACKGROUND OF THE INVENTION

### 1.Field of the invention

This invention relates to a compositon of matter for relieving hemorrhoidal symptoms and swelling. The invention also relates to a method of reducing the swelling and eliminating hemorrhoidal symptoms,almost immediate, by ingesting a prescribed dose of the composition.

### 2.Description of Related Art

A hemorrhoid is an itching,often painfull mass of dilated veins in swollen anal tissue. Hemorrhoids may be categorized as internal or extenal,depending upon whether the internal or external hemorroidal plexus of veins is enlarged. Both types of hemorrhoids are common and are associated with increased pressure in the portal veinous system,such as during pregnancy and by straining during defecation.

Both internal and external hemorrhoids may be quite painful,although internal hemorrhoids typically are not painful until there exsist thrombosis,infection,or erosion of the mucosal surface which overlies the hemorrhoid. Typically,any of these conditions results in bleeding,manifested as blood on the stool,and a feeling of vague anal discomfort,including pruritus and a burning sensation. An internal hemorrhoid may prolapse through the anus. Although the level of psycal discomfort may not increase,one who suffers such a condition will be embarrassed by constant soiling of the underclothing. Further a prolapsed hemorrhoid may become infected or thrombosed,and is susceptible to profuse bleeding upon defecation.

Hemorrhoids can be treated by sizt baths or other forms of moist heat,suppositories,stool softeners,and bed rest.
Various commercially available preparations are intended to be applied to the hemorrhoids to relieve the pain and itch of swollen hemorrhoidal tissue,but such preparations do little to reduce hemorrhoidal symptoms. Other compositions are designed to be ingested or to be applied to hemorrhoids wich are externally accessible. One such composition is disclosed in U.S.A Leptandra Culver's,but the product when fresh may produce bloody stools and possibly abortion. The internal hemorrhoids which have a low degreeof prolapse with pruritus can be treated by injection of sclerosing solutions.

Generally the prolapsed and external hemorrhoids are treated surgically.

### SUMMARY OF THE INVENTION

An object of this invention to provide an edible composition of matter that eliminates hemorrhoidal symptoms and swelling. It is a further object of this invention to provide an edible composition comprising sulfur and cream of tartar for eliminates hemorrhoidal symptoms.

It is another object of this invention to provide a method of eliminating hemorrhoidal symptoms and reabsorbing hemorrhoidal prolapse too.

A still further object of this invention to eliminate hemorroidal symptoms and swelling by ingesting sulfur and crem of tartar containing medicament.

A still object of the invention is to eliminate in a short time all the hemorrhoidal symptoms(itching*burning and swelling) and consequently,eliminate the venous prolapse they have caused,without any side effect,such as intestinal disturbs,for example.

For this goal it has been established that the unfailing formula which eliminates in a short time all the hemorrhoidal symptoms and acts quickly on the swelling,without any side disturb is combined with the following elements* 22 weight percent of sulfur (better flowers of sulfur) and 78 weight percent of cream of tartar.

In accordance with these or other objects,the invention relates to a composition comprising 22 weight percent sulfur and 78 weight percent of crem of tartar,active ingredients for reducing hemorrhoidals. The invention also relates to method of relieving hemorrhoidal symptoms by ingesting a prescribed dose of this composition.

With reference to U.S.A. patent number 4,985,257 Hemorrhoidal treatment and composition it has established,after testing a certain number of persons,that the therapeutically effective dose which doesn't involve any kind of disturb, is the dose described in the method and the percentages of the composition are 22 weight percent of sulfur and 78 weight percent cream of tartar.Thus,it has been noted that same doses,with percentages higher of sulfur may cause an any subject(for example on subject suffering of colitis)a weak slowing down of faeces.

On the contrary,the ingestion of the same doses having lower percentages of sulfur will require a longer period of treatment because recovery and improvement phases will be longer.

### DETAILED DESCRIPTION OF THE INVENTION

This invention is based on the discovery that ingestion of a composition of matter comprising flowers of sulfur and cream of tartar as active ingredients eliminates almost immediate hemorrhoidal symptoms,.Ingestion of a therapeutically effective quantity of this composition eliminates hemorrhoidal symptoms thus reducing the likelihood of permanent hemorrhoidal prolapse and also reabsorbing the existing prolapse in large number of cases.

The invention also is based on the discovery of a particular range of flowers of sulfur and cream of tartar in the composition of matter which,when ingested, eliminates hemorrhoidal symptoms and reduce swelling yet does not produce any undesirable side effects.

The composition of matter this invention comprises flower of sulfur and cream of tartar as the sole necessary therapeutically active ingredients to eliminates hemorrhoidal symptoms and reduce the swelling within a short period.

Depending upon the form in which the composition is made available to the user,pharmaceutically unobjectionable adjuvants such as extenders,bulking agents,flavoring and coloring agents(whether natural or artificial),and emulsifiers or suspending agents,as described below,may,of course,be present with sulfur and cream of tartar in the composition.

The composition of the invention comprises 22 weigth percent sulfur flowers and 78 weight percent of cream of tartar,based on the combined weight of sulfur flowers and cream of tartar.

Cream of tartar also is known by a variety of names,including potassium bitartrate,potassium hidrogen tartrate,and potassium acid tartrate. Cream of tartar exists as colorless crystals or as white crystalline powder. Cream of tartar is soluble in water.

Sulfur is available in a varity of forms,each of which is a crystalline powder suitable for use in the practice of this invention.
1 However,it is preferred that the forms of sulfur commonly called ''pharmaceutical sulfur'',sublimed sulfur(flowers of sulfur)washed sulfur and precipitated sulfur('milk of sulfur') be utilized.

More preferably,sublimed sulfur is utilized as an active ingredient in the composition of the invention. Sulfur is not soluble in water.

The form in which the edible composition is supplied to the consumer is not critical and forms no part the invention. Because the composition is a mixture of two solid,crystalline powders,the composition conveniently is supplied as a homogeneously blended powder of the desired proportion of sulfur and cream of tartar.together with any adjuvants.

Because sulfur is a pale yellow to yellow powder and cream of tartar is colorless white crystal and comprises the mayor fraction of the composition,the composition of the invention is blended powder form typically would be a light yellow compound. the composition of the invention is not completely soluble in water,but,with agitation,will form a suspension which is sufficiently stable for a period which is relatively short,but sufficient to drink the suspension.

The taste of the composition is not unpleasant. However,if desired,flovoring may be added.

In accordance with the method of the invention,a therapeutically dose of the composition is ingested when symptoms of hemorrhoidal suffering are experienced. Typically,a therapeutically daily dose is between about 25 and 30 grams of powdered composition consisting solely of ingredients active in the elimination of the hemorrhoidal swelling i.e.,sulfur and cream of tartar. More preferably a daily dose of between 30 grams in two times.

The dosage may be adjusted by the user to accomodate differences(for example,body weigth,severity of the symptoms)among individuals. Preferably,about half of the therapeutically effective dosage is taken twice daily,preferably at approximately twelve hours intervals. Treatment is continued until complete elimination of hemorrhoidal symptoms and resorption of probable prolapse.

Thus, the therapeutically effective volume of a composition of the invention consisting solely of powdered sulfur and cream of tartar is between two tablespoons daily,where one tablespoon is equivalent to a volume of approximately 10 cubic centimeters. Is better don't exceed with the daily dose(1 or max 2 tablespoons).

The therapeutically effective dosage described herein considers only the weight or volume of the ingredients therapeutically active against hemorrhoidal swelling and symptoms,i.e.only sulfur and cream of tartar.

Skilled practitioners recognize that adjuvants affect the weight or volume of the dosage and the quantity of pharmaceutically and phisiologically unobjectable adjuvants may vary significantly between dosage types.

For example, a pill or tablet compressed for ingestion without chewing typically will have little additional material,and may be significantly more dense than loose powder. Similarly,a therapeutically effective quantity of a powdered composition to which a large quantity of inert material such a flavoring(for example sugar)and coloring agents has been added,will be not only greater in weight but also larger in volume than the active ingredients alone.

It has been discovered that ingestion of a therapeutically quantity of the composition of the invention ,i.e., a composition consisting essentially of 22 weight percent sulfur(better flowers) and 78 weight percent cream of tartar,quickly relieves the burning and itching of painful hemorrhoidal tissue. Not only does the compound promptly relieve these annoying symptoms,but also it subsequently eliminates the swelling of the hemorrhoids,thus reducing the likelihood of thrombosis,erosion of the mucosa over the hemorrhoid,infection,and likelihood that an internal hemorrhoid will become permanently prolapsed. The composition of the invention is able to eliminate the existent prolapse definitively in some cases of hemorrhoids. Use of the product of the invention in accordance with the method described causes reabsorption of existing prolapse.

Further,ingestion of a medicament clearly is more convenient than application of greasy or oily compositions,such as known treatments,to the affected tissues,which typically are not externally visible.

A second dose is taken about 12 hours after the first is taken,if you want.

### EXAMPLE 2

One tablespoon(about15 grams)of the composition described in Example 1 is mixed with about half glass of spring water. A small fraction of cream of tartar dissolves,but the remainder of cream of tartar and all the flowers of sulfurt remain indissolved. However with sufficient stirring,these particles remain suspended in the water for a period sufficient to drink the suspension to relieve the symptoms and the swelling.

### EXAMPLE 3

Twentytwo grams of sulfur,78 grams of cream of tartar,and about 08/10 grams of common sugar or fructose are mixed to form a homogenous blend. When hemorrhoidal symptoms are experienced, about 15 grams(one tablespoon) of the mixture are admixed with about a glass of spring water,
The sugar and small quantity of cream of tartar dissolve,while the remaining cream of tartar and sulfur remain suspended in the water after stirring is stopped for a time sufficient to drink the suspension. A second equal quantity of powder is ingested a second dose about 12 hours after the first ingestion. Hemorrhoidal symptoms are relieved quickly and the swelling is eliminated. One tablespoon after experiencing hemorrhoidal symptoms can be ingested without melt it in the water but ingesting directly.

The next dose won't be necessary if itching and burning will be eliminate at first.

During the therapy for eliminating of possible prolapse a new dose is advisable 24 hours after the previous two doses.

Although preferred embodiments of this invention have been described herein,skilled practitioners recognize that changes and modification may be made without departing from the spirit of the invention,as defined in and limited only by the scope of the appened claims.

## Claims

1. A composition of matter comprising as active ingredients for eliminating hemorrhoidal symptoms and relieving swelling with the following components:22 weight percent sulfur(better 'flowers of sulfur')and 78 weight percent cream of tartar blended together.

2. A composition for the elimination and treatment of internal and external hemorrhoids and for the symptoms (burning-itching-swelling) in accordance with claim 1. wherein said composition is edible and is eaten to effect said elimination and treatment.

3. The composition of claim 1.further comprising pharmaceutically and also unobjectionable adjuvants.

4. A method for eliminate hemorrhoidal symtoms and reducing the swelling comprising ingesting after experiencing hemorrhoidal symptoms a advised effective dose of a composition comprising, as active ingredients for eliminating hemorrhoidal symptoms and reducing swelling,of 22 weight percent sulfur(better 'flowers of sulfur)and 78 weight percent cream of tartar blended together.

5. The method of claim 4.wherein the advised effective but not laxative dose is between about 15 and 30 grams of sulfur and cream of tartar daily.

6. The method of claim 5. wherein the advised effective dose of the composition is ingested in two approximately equal doses daily.

7. The method for the elimination and the treatment of internal and external hemorrhoids and hemorrhoidal symptoms of the human body comprising eating the composition of clain 1.to effect said eliminate and treat.
